# EUROPEAN PATENT APPLICATION

(11) **EP 4 296 300 A1**
(43) Date of publication of application: **27.12.2023**
(21) Application number: 22756258.4
(22) Date of filing: 17.02.2022
(51) Int. Cl.: C08J 7/04, A61K 8/02, A61K 8/73, A61K 8/9794, C08J 7/06

(54) **COATED PARTICLE, METHOD FOR PRODUCING SAME, AND COSMETIC**

(30) Priority: 19.02.2021 JP 2021025176
(71) Applicant: JGC Catalysts and Chemicals Ltd., Kawasaki-shi, Kanagawa 212-0013 (JP)
(72) Inventor: ENOMOTO, Naoyuki, Kitakyushu-shi, Fukuoka 808-0027 (JP); HAMASAKI, Yuichi, Kitakyushu-shi, Fukuoka 808-0027 (JP); SHIMAZAKI, Ikuko, Kitakyushu-shi, Fukuoka 808-0027 (JP)
(74) Representative: Dennemeyer & Associates S.A.
(86) International application number: PCT/JP2022/006346
(87) International publication number: WO 2022/176940

(57) **Abstract**

The present invention relates to coated particles in which vegetable wax is provided on the surface of starch particles. The coated particles have an average particle diameter d₁ of 0.5 to 20 µm and a maximum particle diameter d₂ being less than 30 µm and less than 4.0 times the average particle diameter d₁. The vegetable wax is contained in an amount of 0.5 to 10.0 wt% in the coated particles. Accordingly, particles having excellent feel characteristics and water repellency can be achieved with a natural material having excellent biodegradability. A method for producing coated particles includes: a step of preparing a dispersion that contains 1 to 20 wt% of starch particles; a step of adding vegetable wax to the dispersion and heating and cooling the mixture to precipitate the vegetable wax on the surface of the starch particles; and a step of subjecting this dispersion to solid-liquid separation to obtain coated particles as a solid.

## Description

### TECHNICAL FIELD

The present invention relates to starch particles surface-treated with vegetable wax.

### BACKGROUND ART

Currently, petroleum-derived synthetic polymers (plastics) are used in various industries. Synthetic polymers are often developed for long-term stability and are not degraded in natural environments. Therefore, various environmental problems have occurred. For example, plastic products that have flowed into the water environment are accumulated for a long period, and marine and lake ecosystems are greatly affected. In recent years, microplastics having a length of 5 mm or less to the nm level have become a major problem. Examples of the microplastics include fine particles contained in cosmetics and the like, small lumps of pre-processed plastic resin, and things obtained as a result of a large product being finely divided while floating in the sea.

In recent years, plastic particles of the several hundred µm class (for example, polyethylene particles) have been blended in cosmetics in order to improve the feel characteristics of cosmetics. Plastic particles, which have a low true specific gravity, are difficult to remove in a sewage treatment plant and easily flow into rivers, oceans, ponds, and the like. Furthermore, plastic particles easily adsorb chemical substances such as insecticides and thus have a risk of affecting the human body through bioconcentration. This is also pointed out by the United Nations Environmental Program and the like, and countries and various industry groups are studying the regulation.

In addition, interest in natural cosmetics and organic cosmetics has increased, and guidelines on natural and organic index display of cosmetics (ISO 16128) have been established. According to the guidelines, a raw material in a product is classified into, for example, a natural raw material, a naturally-derived raw material, and a non-natural raw material, and an index is calculated based on the content of each raw material. In the future, the index will be displayed on a product in accordance with the guidelines, and thus it is expected that a naturally-derived raw material and further a natural raw material will be required.

From such a background, attention has focused on biodegradable plastics which are decomposed into water and carbon dioxide by microorganisms or the like in a natural environment and incorporated into the carbon cycle in nature. In particular, cellulose particles, which are a plant-derived natural raw material, do not float on water even when flowing out to the environment and have good biodegradability, so that there is little concern for causing environmental problems. For example, it is known to neutralize with an acid a copper ammonia solution in which cellulose is dissolved to obtain 9 to 400 nm spherical regenerated cellulose particles (see, for example, JP-T-2008-84854). It is also known to spray a cellulose solution to form droplets in a gas phase and bring the droplets into contact with a coagulation liquid to obtain spherical regenerated cellulose particles (see, for example, JP-A-2013-133355). In these example, cellulose particles are prepared using type II crystalline cellulose obtained by a process of performing intentional chemical modification. Such regenerated cellulose particles are classified as a naturally-derived raw material according to the definition of the above-described guidelines. It is also known to prepare porous cellulose particles having a type I crystal form by granulating and drying cellulose dispersed in an organic solvent by a spray drying method (see, for example, JP-A-2 -84401). It is also known that when crystalline cellulose is surface-treated with metal soaps or hydrogenated lecithin, a cosmetic powder having excellent water repellency and oil repellency and excellent soft feel to the skin is obtained (see, for example, JP-A-2003-146829).

### SUMMARY OF INVENTION

### PROBLEMS TO BE SOLVED BY INVENTION

In "Note on substance identification and the potential scope of a restriction on uses of 'microplastics' Version 1.1" issued on October 16, 2018, the European Chemicals Agency presented a view that microplastics are premised on being a synthetic polymer, and naturally-derived cellulose, starch, and the like having biodegradability should not be regarded as microplastics.

However, the cellulose in the patent literatures is a trans-type polysaccharide that contains glucose molecules as a constituent unit and is insoluble to water. Therefore, it is considered that cellulose is not highly degradable in nature. The definition of biodegradability is also still not clear, and there remains a concern that cellulose will become a substance to be regulated.

Therefore, an object of the present invention is to achieve particles having excellent feel characteristics and water repellency using a water-soluble material.

### SOLUTION TO PROBLEMS

In the present invention, the surface of starch particles was treated with vegetable wax to form coated particles. Both starch and cellulose are polysaccharides having glucose as a constituent unit, but both are isomers. That is, starch is a cis type, and cellulose is a trans type. This difference is related to biodegradability and the presence or absence of water solubility. Cosmetics in which water-soluble starch particles are blended have little concern for causing environmental problems and further can have feel characteristics similar to those of known plastic beads.

The coated particles according to the present invention are starch particles surface-treated with rice bran wax. The coated particles have an average particle diameter d₁ of 0.5 to 20 µm and a maximum particle diameter d₂ being less than 30 µm and within 4.0 times the average particle diameter d₁. The vegetable wax is contained in an amount of 0.5 to 10.0 wt% in the coated particles.

A method for producing coated particles according to the present invention includes: a step of preparing a dispersion that contains 1 to 20 wt% of starch particles; a step of adding vegetable wax to the dispersion, heating the mixture, and cooling the mixture to precipitate a vegetable wax component on a surface of the starch particles; and a step of obtaining coated particles as a solid from the dispersion by solid-liquid separation. In this method, the vegetable wax is preferably added in an amount of 0.5 to 11.0% by mass with respect to the mass of the starch particles contained in the dispersion.

As the vegetable wax, rice bran wax is preferably used. Furthermore, it is preferable to use starch particles having an amylopectin content of 90 wt% or more. Also, the content of globulin as protein is preferably 0.02 wt% or less.

Cosmetics can be produced by blending the above-described coated particles.

### DESCRIPTION OF EMBODIMENTS

The present invention relates to coated particles in which vegetable wax is provided on the surface of starch particles. The coated particles are particles obtained by surface-treating starch particles with vegetable wax and contain 0.5 to 10.0 wt% of the vegetable wax. That is, the weight of the vegetable wax is equivalent to 0.5 to 10.0% of the weight of the coated particles. When the weight of the vegetable wax is less than 0.5%, desired water repellency cannot be imparted. When it exceeds 10.0%, the coated particles may adhere to one another to form a large aggregate. The weight is more preferably 1.0 to 5.0%. The surface of the starch particles does not need to be completely covered with the vegetable wax, and a part of the surface of the starch particles may be exposed. When the vegetable wax having the above-described ratio to the weight of the coated particles is detected, the effect of water repellency is exerted. The weight of the vegetable wax can be quantified by infrared absorption spectrum measurement.

The coated particles have an average particle diameter d₁ of 0.5 µm or more and 20 µm or less and a maximum particle diameter d₂ being less than 30 µm and 4.0 times or less the average particle diameter d₁. The average particle diameter d₁ affects the feel characteristics of cosmetics. When it is less than 0.5 µm, feel characteristics such as rolling feeling, sustainability of rolling feeling, and uniform spreadability significantly deteriorate. The average particle diameter d₁ is preferably 1.5 µm or more, more preferably 2 µm or more, and optimally 5 µm or more. When it is more than 20 µm, roughness is felt, and soft feeling and moist feeling deteriorate. The average particle diameter d₁ is preferably 15 µm or less and more preferably 10 µm or less. Also, when the maximum particle diameter d₂ is 30 µm or more, roughness is felt, and soft feeling and moist feeling deteriorate. When the maximum particle diameter exceeds 4.0 times the average particle diameter, uniform spreadability deteriorates. Here, the average particle diameter d₁ is a median value obtained from the particle size distribution of the particles, and the largest particle diameter detected in the particle size distribution is the maximum particle diameter d₂.

Examples of typical vegetable wax include rice bran wax, carnauba wax, candelilla wax, and wood wax. Carnauba wax and rice bran wax, which have a relatively high melting point, are preferable. Furthermore, rice bran wax which is also used for food is most preferable. Rice bran wax is mainly formed of a higher alcohol ester component and a fatty acid component and does not contain a free alcohol component having odor.

Furthermore, it is preferable to use starch particles having an amylopectin content of 90 wt% or more. In general, starch is formed of amylose and amylopectin. Amylose has a structure in which glucose molecules are linearly connected. Amylopectin is large in molecular weight and has a structure in which amylose is branched and connected. The larger the content of amylose, the higher the swellability of the starch particles, which causes stickiness when blended in cosmetics. Therefore, the content of amylose is preferably less than 10 wt%. The content of amylopectin is preferably as large as possible. The content of amylopectin is more preferably 95 wt% or more and most preferably 98 wt% or more. The content of amylopectin can be measured by a gel filtration method, an iodine colorimetric method, a two-wavelength measurement method, or the like.

The starch particles contain proteins derived from the raw material. Among the proteins, globulin has strong allergen activity and is regarded as a causative substance of rice allergy. Therefore, the content of globulin is preferably 0.10 wt% or less. The content of globulin is suitably 0.05 wt% or less and further suitably 0.02 wt% or less. The content of globulin can be reduced by enzymatically treating the raw material (generally starch grains) of the starch particles. Other examples include an immersion treatment in a saline solution, a dilute acid solution, or an alkaline aqueous solution, and a high pressure treatment (for example, 100 to 400 MPa). These treatments may be combined.

The coated particles preferably have a sphericity of 0.85 or more, that is, a spherical shape. The higher the sphericity, the better the rollability of cosmetics. The sphericity is particularly preferably 0.90 or more. The sphericity is determined from a scanning electron microscope photograph by an image analysis method.

The particle variation coefficient (CV) of the coated particles is preferably 50% or less. When the particle variation coefficient exceeds 50%, uniform rollability may not be obtained. The particle variation coefficient is preferably 40% or less and particularly preferably 30% or less. It is noted that although the smaller particle variation coefficient is more suitable, it is industrially difficult to obtain particles having a narrow distribution. With a particle variation coefficient of about 3% or more, production can be performed without any particular problem.

The vegetable wax present on the surface of the coated particles has little influence on the particle shape. Therefore, it is desirable that the starch particles also have the above-described characteristics of the coated particles.

### <Method for producing starch particles>

Next, a method for producing coated particles will be described. Here, coated particles obtained by surface-treating starch particles with rice bran wax will be described in detail.

First, starch particles are dispersed in a solvent to prepare a dispersion in which the solid content concentration of the starch particles is 1 to 20 wt% (first step). To this dispersion, rice bran wax is added and heated. Accordingly, the rice bran wax is dissolved in the solvent. Next, the dispersion is cooled to precipitate the dissolved rice bran wax on the surface of the starch particles (second step). Next, the dispersion is subjected to solid-liquid separation to obtain coated particles as a solid (third step). The starch particles to be used here may be prepared by a known method. Also, a solvent having a boiling point higher than the melting point of rice bran wax is used.

Hereinafter, each step will be described in detail.

### [First step]

First, a dispersion of starch particles is prepared. The solid content concentration of the starch particles is adjusted to a range of 1 to 20 wt%. When the solid content concentration of the dispersion exceeds 20 wt%, the starch particles are likely to be aggregated. Therefore, the coating state of the rice bran wax may not be uniform in the subsequent step. In that case, desired water repellency cannot be obtained. When the solid content concentration is less than 1 wt%, the yield of coated particles is small and poor in economic efficiency. As the solvent of the dispersion, there is used a solvent in which rice bran wax is dissolved by heating. The solvent is preferably an organic solvent. In particular, the solvent is preferably an alcohol-based solvent and suitably isopropyl alcohol (IPA).

### [Second step]

To this dispersion, rice bran wax is added and heated so that the rice bran wax is dissolved in the solvent. Heating is performed at not lower than 78°C which is the melting point of rice bran wax. The temperature is preferably lower than 90°C but may be appropriately determined depending on a used solvent. When the temperature is low, rice bran wax is not dissolved or is insufficiently dissolved, and the coating state may not be uniform. When the temperature is 90°C or higher, heating takes time, and economic efficiency is poor.

The mass of the rice bran wax to be added is preferably 0.5 to 11.0% of the mass of the starch particles. When the weight of the vegetable wax is less than 0.5%, desired water repellency cannot be imparted. In order for the coated particles to contain about 10 wt% of vegetable wax, about 11% of rice bran wax is preferably added. However, when it exceeds 11.0%, the coated particles may adhere to one another to form a large aggregate. The weight is more preferably 1.0 to 5.0%.

Next, this dispersion is cooled to precipitate the rice bran wax on the surface of the starch particles. The cooling temperature may be such that the rice bran wax is precipitated, and is preferably lower than 40°C. When it is 40°C or higher, the dissolved rice bran wax may not be sufficiently precipitated. The amount of the remaining dissolved rice bran wax increases, and the dissolved rice bran wax is discharged outside the system by solid-liquid separation in the subsequent step. Therefore, the starch particles are insufficiently coated, and sufficient water repellency cannot be obtained. Also, in the case of cooling to lower than 20°C, the time required for cooling is lengthened, and thus economic efficiency is poor.

### [Third step]

Next, the solid content is separated from the dispersion of coated particles using a method such as known filtration or centrifugation. Accordingly, a cake-like substance of coated particles is obtained.

After this step, a drying step of heating under normal pressure or reduced pressure may be provided in order to evaporate the solvent remaining in the cake-like substance. Further, the dried solid is disintegrated. Accordingly, a dry powder of coated particles having an average particle diameter of 0.5 to 20 µm is obtained.

### <Cosmetics>

Cosmetics can be prepared by blending the above-described coated particles and various cosmetic components. According to such cosmetics, there can be simultaneously obtained rolling feeling, sustainability of rolling feeling, and uniform spreadability which are similar to those of inorganic particles (silica particles) of a single component as well as soft feeling and moist feeling which are similar to those of plastic beads. That is, it is possible to satisfy typical feel characteristics required of feel improvers for cosmetics.

Specific cosmetics are exemplified for each classification in Table 1. Such cosmetics can be produced by a known general method. Cosmetics are used in various forms such as a powder form, a cake form, a pencil form, a stick form, a cream form, a gel form, a mousse form, and a liquid form.

Representative classifications and components as various cosmetic components are exemplified in Table 2. Furthermore, cosmetic components listed in the Quasi-drug raw material standard 2006 (issued by Yakuji Nippo, Limited, June 16, 2006), the International Cosmetic Ingredient Dictionary and Handbook (issued by The Cosmetic, Toiletry, and Fragrance Association, Eleventh Edition 2006), and others may be blended.

**[Table 1]**

| Cosmetics for washing | Soap, cleansinng foam, makeup removing cream |
|---|---|
| Skin care cosmetics | Moisture retention and rough skin preventbn, acne, keratin care, massage, wrinkles or bags treatment, dullness or dark circles treatm ent, UV care, skin-whitening, antioxidative care |
| Base makeup cosmetics | Pow der foundation, liquid foundation, cream foundation, mousse foundation, pressed pow der, makeup base |
| Point makeup cosmetics | Eyeshadow, eyebrow, eyeliner, mascara, lipstick |
| Hairr care cosmetics | Hair growth, dandruff prevention, itch prevention, washing, conditioning and hair styling, permanent and waving, hair cobring and hair bleaching |
| Body care cosmetics | Washing, sunburn prevention, rough hand prevention, slimming, bbod circulation in provement, itch suppression, body odor prevention, antiperspration, body hair care, repellent, body powder |
| Fragrance cosmetics | Perfume, eau de parfum, eau de toilette, eau de cobgne, shower cobgne, solid perfume, body lotion, bath oil |
| Oral care products | Toothpaste, mouthwash |

**[Table 2]**

| Classifications | Component examples |
|---|---|
| Oils and fats | Olive oil, rapeseed oil, beef talbw, jojoba oils |
| Waxes | Carnauba wax, candelilla wax, beeswax |
| Hydrocarbons | Paraffin, squalane, synthetic or vegetable squalane, *α*-olefin oligomer, microcrystalline wax, pentane, hexane |
| Fatty acids | Stearic acid, myristic acid, oleic acid, α-hydroxy acid |
| Alcohols | Isostearyl alcohol, octyl dodecanol, lauryl alcohol, ethanol, isopropanol, butyl alcohol, myristyl alcohol, cetanol, stearyl alcohol, behenyl alcohol |
| Polyalcohols | Ethylene glycol, triethylene glycol, polyethylene glycol, propylene glycol, glycerin, diglycerin, 1,3-butylene glycol |
| Esters | Akyl glyceryl ethers, isopropylmyristate, isopropyl palmitate, ethyl stearate, ethyloleate, cetyllaurate, decyl oleate |
| Saccharides | Sorbitol, glucose, sucrose, trehabse, mixed isomerized sugar, pullulan |
| Silicone oil | Methylpolysibxane, methyl hydrogen polysibxane, methyl phenyl silicone oil, various modified silicone oils, cyclib dimethyl silicon oil |
| Silicone gel | Silicone gel cross linked with silicone-base and/or other organic compound |
| Surfactant | Nonionic, cationic, anionic |
| Fluorine oil | Perfluoropolyether |
| Varous polymers | Gum arabic, carrageenan, agar, xanthan gum, gelatin, alginic acid, pullulan, abumin, guar gum, carboxy vinylpolymer, polyvinyl alcohol, cellubse and its derivative, polyacrylib acid amide, sodium polyacrylate |
| UV protective agent | Cinnamic acid-based such as octylparam ethoxycinnamate, salicylic acid-based, benzoic acid ester-based, urocanic acid-based, benzophenone-based |
| Inorganic compound | Titanium oxide, zinc oxide, aluminum oxide, aluminum hydroxide, red iron oxide, yellow iron oxide, black iron oxide, cerium oxide, zirconium oxide, silica, mica, talc, sericite, boron nitride, barium sulfate, titanated mica having pearl gloss, (including composites thereof) (may be surface-treated (with silicone, fluorine, metal soap, or the like) as necessary) |
| Resin particles | Methylpolyacrylate, nylon, silicone resin, silicone rubber, polyethylene, polyester, polyurethane |
| Skin- lightening component | Arbutin, kojic acid, vitamin C, linolic acid, linoleic acid, lactic acid, tranexamic acid, ascorbic acid derivative (sodium ascorbate, ascorbic acid phosphate magnesium, ascorbyl di-palmitate, glucoside ascorbate, or the like), plant extract (placenta extract, sulfur, oil-soluble licorice extract, muberry extract, or the like) |
| Roughskin improving component | Various vitamins, carotenoid, flavonoid, tannin, caffeic derivative, lignan, saponin, retinoic acid and retinoic acid structure analog, N-acetyl glucosamine, ε-aminocaproic acid, α-hydroxy acid, glycyrrhizic acid, biopolymers (sodium hyaluronate, collagen, elastin, chitin or chitosan, sodium chondroitin sulfate), amino acid, betaine, ceramide, sphingolipid, cholesterol and its derivative |
| Other components | Bactericide and preservative, antioxidant, modified or un modified clay mineral, various organic pigments and dyes, solvent, flavor, water |

### EXAMPLES

Hereinafter, examples of the present invention will be specifically described.

### [Example 1]

First, a starch dispersion is prepared. To 4750 g of pure water, 250 g of Motil B (registered trademark) manufactured by Joetsu Starch Co., Ltd., as starch grains, was added. The obtained suspension was heated and stirred (120°C, 16 hours). Accordingly, a starch dispersion (solid content concentration: 5 wt%) was obtained. Two hundred grams of this dispersion was added to a mixed solution of 3346 g of heptane (manufactured by Kanto Chemical Co., Inc.) as a nonaqueous solvent and 25 g of a surfactant (AO-10V manufactured by Kao Corporation). This mixed solution was stirred at 10,000 rpm for 10 minutes using an emulsifying and dispersing machine (T.K. Robomix manufactured by Primix Corporation). Accordingly, the solution was emulsified to obtain an emulsion that contains emulsified droplets. This emulsion was allowed to stand in a freezer at -25°C for 72 hours to freeze water in the emulsion droplets. Thereafter, the temperature was raised to normal temperature to thaw the frozen water. This was filtered through quantitative filter paper (No. 2 manufactured by Advantec Toyo Kaisha, Ltd.) using a Buchner funnel (3.2 L, manufactured by Sekiya Chemical Glass Apparatus Co., Ltd.). Thereafter, washing with 2500 g of heptane was repeated three times to remove the surfactant. The thus obtained cake-like substance was dried at 60°C for 12 hours. This dried powder passed through a 250 mesh sieve (JIS test standard sieve) to obtain starch particles.

Next, 9 g of the starch particles was suspended in 81 g of IPA (2-propanol manufactured by Kanto Chemical Co., Inc.). This suspension was treated in a bath type ultrasonic device (manufactured by SSD Co., Ltd.) for 10 minutes to prepare an IPA dispersion of starch particles (solid content concentration: 10 wt%).

To 90 g of this dispersion, 0.14 g of rice bran wax (NC-1720 manufactured by Cerarica Noda Co., Ltd.) was added, and the temperature was raised to 78°C under stirring. Furthermore, this mixed liquid was stirred for 10 minutes and cooled to 30°C over 150 minutes. This solution was filtered through quantitative filter paper (No. 2 manufactured by Advantec Toyo Kaisha, Ltd.) using a Buchner funnel (3.2 L, manufactured by Sekiya Chemical Glass Apparatus Co., Ltd.). The thus obtained cake-like substance was dried at 80°C for 16 hours. This dried powder passed through a 250 mesh sieve (JIS test standard sieve) to obtain a powder of coated particles.

The preparation conditions of coated particles are summarized in Table 3. Also, the physical properties of the powder of coated particles were measured by the following method. Other Examples and Comparative Examples were also measured in the same manner. The results are illustrated in Table 4.

### (1) Average particle diameter, maximum particle diameter, and particle variation coefficient

The particle size distribution of the coated particles was measured using a laser diffraction method. From this particle size distribution, a median value was determined and defined as the average particle diameter d₁. The largest particle diameter detected in the particle size distribution was defined as the maximum particle diameter d₂. Further, the standard deviation σ and the population mean µ were determined from the particle size distribution (population) to obtain the particle variation coefficient (CV = σ/µ). In Table 4, the values are expressed as a percentage. In addition, the maximum particle diameter d₂ was divided by the average particle diameter d₁ to obtain the ratio (d₂/d₁) between the maximum particle diameter and the average particle diameter. Here, the particle size distribution was measured using LA-950v2 manufactured by Horiba, Ltd.

### (2) Content of rice bran wax

An absorption peak attributed to a carbonyl group contained in a higher alcohol ester which is a main component of rice bran wax is around 1700 cm⁻¹. Therefore, the content of rice bran wax was determined based on the value around 1700 cm⁻¹ of the IR spectrum measured using an infrared absorption spectrometer.

The powder of rice bran wax and the powder of starch particles are previously mixed uniformly using an agate mortar. At this time, samples are prepared such that the ratio of rice bran wax is 0.5 wt%, 5 wt%, 10 wt%, and 20 wt%. Twenty milligrams of each sample powder is molded into a 20 ϕ disk. This was placed in an IR cell connected to a vacuum line and subjected to a vacuum evacuation treatment at 70°C for 1 hour to remove adsorbed moisture. After the vacuum evacuation treatment, the temperature is lowered to 25°C, and the IR spectrum of the sample disk is measured with an infrared absorption spectrometer (FT/IR-4600 manufactured by JASCO Corporation). Using the absorption peak intensity around 1700 cm⁻¹ obtained in each sample, a calibration curve of the content of rice bran wax and the peak intensity is obtained.

On the other hand, 20 mg of the powder of coated particles was molded into a 20 ϕ disk. Using this disk, an IR spectrum was measured in the same manner as described above, and the absorption peak intensity around 1700 cm⁻¹ was obtained. This value was compared with the previously prepared calibration curve to determine the content of rice bran wax contained in the coated particles.

### (3) Content of amylopectin

Using an amylose/amylopectin analysis kit (manufactured by Biocon (Japan) Ltd.), the total starch amount and the amylose amount were measured according to a procedure defined by the kit. The content (wt%) of amylopectin was obtained according to " {1 - (amylose amount/total starch amount)} × 100".

### (4) Globulin amount

The globulin residual amount was measured using an SDS-polyacrylamide gel electrophoresis method. First, 2 g of sodium dodecyl sulfate (manufactured by FUJIFILM Wako Pure Chemical Corporation), 24 g of urea (manufactured by FUJIFILM Wako Pure Chemical Corporation), 10 g of glycerin, and 0.76 g of tris(hydroxymethyl)aminomethane (manufactured by Tokyo Chemical Industry Co., Ltd.) were dissolved with distilled water. This solution was adjusted to pH 6.8 with a 1 N aqueous hydrochloric acid solution. Further, 2.5 g of 2-mercaptoethanol (manufactured by FUJIFILM Wako Pure Chemical Corporation) was added to make 100 mL, and this was used as a protein extract. One gram of the powder of coated particles obtained in the example was added to 0.7 mL of the protein extract. The mixture was stirred and allowed to stand for 24 hours. Thereafter, the supernatant was separated by centrifugation. This supernatant in an amount of 0.01 mL was charged into PGME gel (manufactured by Funakoshi Co., Ltd.), and electrophoresis was performed. Thereafter, this gel was immersed in a CBB staining reagent (manufactured by BioDynamics Laboratory Inc.) to be stained. The stained gel was subjected to image analysis (Lumino image analyzer LAS-100 plus manufactured by Fujifilm Corporation, automatic quantification with Image Gauge manufactured by Fujifilm Corporation) to calculate the globulin residual amount.

### (5) Sphericity

A photograph is taken with a transmission electron microscope (H-8000 manufactured by Hitachi, Ltd.) at a magnification of 2000 to 250000 to obtain a photographic projection view. From this photographic projection view, 50 particles were arbitrarily selected. The maximum diameter D_{L} of each particle and the minor axis D_{S} orthogonal thereto were measured, and the ratio (D_{S}/D_{L}) was obtained. The average value thereof was defined as the sphericity.

### (6) Water repellency

The powder of coated particles in an amount of 0.5 g was poured in 30 ml of water, left to stand for 1 hour, and then visually observed. When the powder as a sample was present on the water surface and was not sunk in water, it was determined as "Good". When the powder was sunk in water, it was determined as "Poor".

### [Example 2]

A powder of coated particles was obtained in the same manner as in Example 1, except that the amount of rice bran wax added to 90 g of the IPA dispersion (solid content concentration: 10 wt%) of starch particles was changed to 0.72 g.

### [Example 3]

A starch dispersion (solid content concentration: 5 wt%) was prepared in the same manner as in Example 1. Forty grams of this dispersion was diluted with 160 g of pure water, and the solid content concentration was adjusted to 1 wt%. Two hundred grams of this diluted dispersion was added to a mixed solution of 3346 g of heptane (manufactured by Kanto Chemical Co., Inc.) and 25 g of a surfactant (AO-10V manufactured by Kao Corporation). Subsequently, an emulsion was prepared under the same emulsification conditions as in Example 1. The emulsion was heated at 60 °C for 16 hours to dehydrate emulsion droplets. After the dehydration, the emulsion was cooled and stored at 2 °C for 16 hours. Furthermore, this was filtered through quantitative filter paper (No. 2 manufactured by Advantec Toyo Kaisha, Ltd.) using a Buchner funnel (3.2 L, manufactured by Sekiya Chemical Glass Apparatus Co., Ltd.). Thereafter, a powder of coated particles was obtained in the same manner as in Example 1.

### [Example 4]

A starch dispersion (solid content concentration: 5 wt%) was prepared in the same manner as in Example 1, and 200 g of this dispersion was added into a mixed liquid of 3346 g of heptane and 25 g of a surfactant (AO-10 V). Thereafter, coated particles were prepared in the same manner as in Example 3. However, the rotation speed of the emulsifying and dispersing machine in the emulsification step was changed to 800 rpm, and the heating time of the emulsion in the dehydration step was changed to 24 hours.

### [Example 5]

In this example, 125 g of Motil B manufactured by Joetsu Starch Co., Ltd. and 125 g of Fine Snow (amylopectin derived from nonglutinous rice: 80%) manufactured by Joetsu Starch Co., Ltd. were mixed to make starch grains. Otherwise, a powder of coated particles was obtained in the same manner as in Example 1.

### [Example 6]

In this example, the globulin amount of starch grains was reduced by an alkali immersion treatment and an enzyme treatment. The starch grains were used to prepare starch particles. First, 1 kg of Motil B was added to 4 kg of pure water, and a 5 wt% aqueous sodium hydroxide solution was added to the obtained suspension to adjust the pH to 9.0 (alkali immersion treatment). Furthermore, 10 g of an enzyme (Aroase AP-10 manufactured by Yakult Pharmaceutical Industry Co., Ltd.) was added, and the mixture was stirred at room temperature for 24 hours (enzyme treatment). Subsequently, this solution was filtered through quantitative filter paper (No. 2 manufactured by Advantec Toyo Kaisha, Ltd.) using a Buchner funnel (3.2 L, manufactured by Sekiya Chemical Glass Apparatus Co., Ltd.). Further, the residual enzyme and the decomposed protein were removed by washing with pure water. The thus obtained cake-like substance was dried at 60°C for 12 hours. This dried powder passed through a 250 mesh sieve (JIS test standard sieve). Accordingly, a powder of starch with reduced globulin was obtained. This powder in an amount of 250 g was suspended in 4750 g of pure water. This suspension was heated and stirred (120°C, 16 hours) to prepare a starch dispersion (solid content concentration: 5 wt%). Thereafter, a powder of coated particles was obtained in the same manner as in Example 1. The globulin residual amount of the coated particles obtained in this example was 0.02 wt%.

### [Example 7]

A powder of coated particles was obtained in the same manner as in Example 1, except that the amount of rice bran wax added to 90 g of the IPA dispersion (solid content concentration: 10 wt%) of starch particles was changed to 0.05 g.

### [Comparative Example 1]

No rice bran wax was added to the IPA dispersion (solid content concentration: 10 wt%) of starch particles. Otherwise, a powder of coated particles was prepared in the same manner as in Example 1.

### [Comparative Example 2]

A powder of coated particles was obtained in the same manner as in Example 1, except that the amount of rice bran wax added to 90 g of the IPA dispersion of starch particles was changed to 1.35 g.

### [Comparative Example 3]

In this comparative example, a starch dispersion having a solid content concentration of 20 wt% was prepared. That is, 1000 g of Motil B was suspended in 4000 g of pure water to prepare a dispersion having a solid content concentration of 20 wt%. Two hundred grams of this dispersion was added into a mixed liquid of 3346 g of heptane and 25 g of a surfactant (AO-10V). Thereafter, starch particles were prepared in the same manner as in Example 4.

**[Table 3]**

| | Preparation method of dispersion of starch particles | | | | | | Dispersion of starch particles | | Vegetable wax | |
|---|---|---|---|---|---|---|---|---|---|---|
| | Dispersion of starch | | Emulsification conditions | | Dehydration (or freezing) conditions | | Concent ration | Solvent | Type | Added amount (to starch) |
| | Type | Solid content concentr ation | Emulsification dispersion rate | Emulsifi cation time | Condition | Time | | | | |
| | | [%] | [rpm ] | [min.] | [°C] | [Hr.] | [%] | | | [%] |
| Example 1 | (1) | 5 | 10000 | 10 | Freezing; -25 | 72 | 10.0 | IPA | Rice bran wax | 1.5 |
| Example 2 | (1) | 5 | 10000 | 10 | Freezing; -25 | 72 | 10.0 | IPA | Rice bran wax | 8.0 |
| Example 3 | (1) | 1 | 10000 | 10 | Heating; 60 | 16 | 10.0 | IPA | Rice bran wax | 1.5 |
| Example 4 | (1) | 5 | 800 | 10 | Heating; 60 | 24 | 10.0 | IPA | Rice bran wax | 1.5 |
| Example 5 | (1) + (3) | 5 | 10000 | 10 | Freezing; -25 | 72 | 10.0 | IPA | Rice bran wax | 1.5 |
| Example 6 | (2) | 5 | 10000 | 10 | Freezing; -25 | 72 | 10.0 | IPA | Rice bran wax | 1.5 |
| Example 7 | (1) | 5 | 10000 | 10 | Freezing; -25 | 72 | 10.0 | IPA | Rice bran wax | 0.5 |
| Comparative Example 1 | (1) | 5 | 10000 | 10 | Freezing; -25 | 72 | 10.0 | IPA | - | 0.0 |
| Comparative Example 2 | (1) | 5 | 10000 | 10 | Freezing; -25 | 72 | 10.0 | IPA | Rice bran wax | 15.0 |
| Comparative Example 3 | (1) | 20 | 800 | 10 | Heating; 60 | 24 | 10.0 | IPA | Rice bran wax | 1.5 |

| | | | | | | | | | | |
|---|---|---|---|---|---|---|---|---|---|---|
| (1) : Motil B (amybpectin 100%, derived from glutinous rice) manufactured by Joetsu Starch Co., Ltd. (2): Pow der obtained by reducing globulin residual amount of (1) (3): Fine Snow (amybpectin 80%, derived from nong lutinous rice) manufactured by Joetsu Starch Co., Ltd. | | | | | | | | | | |

**[Table 4]**

| | Physical properties of coated particles | | | | | | | | |
|---|---|---|---|---|---|---|---|---|---|
| | Amybpectin content (wt%) in starch particles | Content of wt% | Average particle (d₁) *µ*m | Maximum particle (d₂) *µ*m | d₂/d₁ | Particle variation (CV) % | Globulin amount wt% | Sphericity | Water repe llen |
| Example 1 | 100 | 1.5 | 6.3 | 15.2 | 2.4 | 34.0 | 0.20 | 0.88 | Good |
| Example 2 | 100 | 7.3 | 6.3 | 15.2 | 2.4 | 34.0 | 0.20 | 0.88 | Good |
| Example 3 | 100 | 1.4 | 1.5 | 4.5 | 3.0 | 29.0 | 0.20 | 0.95 | Good |
| Example 4 | 100 | 1.5 | 18.9 | 29.9 | 1.6 | 41.0 | 0.20 | 0.92 | Good |
| Example 5 | 90 | 1.4 | 6.3 | 15.2 | 2.4 | 34.0 | 0.20 | 0.88 | Good |
| Example 6 | 100 | 1.4 | 6.3 | 15.2 | 2.4 | 34.0 | 0.02 | 0.88 | Good |
| Example 7 | 100 | 0.5 | 6.3 | 15.2 | 2.4 | 34.0 | 0.20 | 0.88 | Good |
| Comparative Example 1 | 100 | 0.0 | 6.3 | 15.2 | 2.4 | 34.0 | 0.20 | 0.88 | Poor |
| Comparative Example 2 | 100 | 13.0 | 6.3 | 15.2 | 2.4 | 34.0 | 0.20 | 0.88 | Good |
| Comparative Example 3 | 100 | 1.5 | 29.0 | 102.3 | 3.5 | 55.0 | 0.20 | 0.85 | Good |

### [Feel characteristics of powder of coated particles]

Next, the feel characteristics of the powders obtained in Examples and Comparative Examples were evaluated. Each powder was subjected to a sensory test by 20 expert panelists, and a hearing survey was conducted on seven evaluation items of smooth feeling, moist feeling, rolling feeling, uniform spreadability, adhesion to the skin, sustainability of rolling feeling, and soft feeling. Evaluation points of the panelists based on evaluation point criteria (a) were summed up, and the feel characteristics were evaluated based on evaluation criteria (b). The results are illustrated in Table 5. As a result, it was found that the powder of each Example was extremely excellent as a feel improver for cosmetics, but the powders of Comparative Examples were not suitable as a feel improver.

### Evaluation point criteria (a)

5 points: Very good
4 points: Good
3 points: Average
2 points: Poor
1 point: Very poor

### Evaluation criteria (b)

Excellent: Total points are 80 or more.
Good: Total points are 60 or more and less than 80.
Fair: Total points are 40 or more and less than 60.
Poor: Total points are 20 or more and less than 40.
Very Poor: Total points are less than 20.

**[Table 5]**

| Evaluation sample | Smooth feeling | Moist feeling | Roling feeling | Uniform spreadability | Adhesion to skin | Susta inability of rolling feeling | Soft feeling |
|---|---|---|---|---|---|---|---|
| Example 1 | Excellent | Excellent | Excellent | Good | Good | Excellent | Excellent |
| Example 2 | Excellent | Excellent | Excellent | Good | Good | Excellent | Excellent |
| Example 3 | Good | Excellent | Good | Good | Excellent | Good | Excellent |
| Example 4 | Excellent | Fair | Excellent | Fair | Fair | Excellent | Fair |
| Example 5 | Excellent | Excellent | Excellent | Good | Good | Excellent | Excellent |
| Example 6 | Excellent | Excellent | Excellent | Good | Good | Excellent | Excellent |
| Example 7 | Excellent | Good | Excellent | Good | Good | Excellent | Good |
| Comparative Example 1 | Excellent | Good | Excellent | Fair | Good | Excellent | Good |
| Comparative Example 2 | Excellent | Excellent | Fair | Fair | Excellent | Poor | Excellent |
| Comparative Example 3 | Excellent | Very Poor | Excellent | Very Poor | Very Poor | Excellent | Very Poor |

### [Use feeling of powder foundation]

Powder foundation was prepared with the powder of coated particles so as to have the blending ratios (wt%) illustrated in Table 6. That is, the powder of each example as the component (1) was put in a mixer together with the components (2) to (9), and the mixture was stirred and uniformly mixed. Next, the cosmetic components (10) to (12) were put into the mixer, and the mixture was stirred and further uniformly mixed. After the obtained cake-like substance was subjected to a disintegration treatment, about 12 g of the disintegrated substance was taken and put in a 46 mm × 54 mm × 4 mm rectangular metal dish and press-molded. The thus obtained powder foundation was subjected to a sensory test by 20 expert panelists. A hearing survey was conducted on six evaluation items of uniform stretch, moist feeling, and smoothness during application to the skin as well as uniformity of the cosmetic film, moist feeling, and softness after application to the skin. Evaluation points of the panelists based on the above-described evaluation point criteria (a) were summed up, and the use feeling of foundation was evaluated based on the above-described evaluation criteria (b). The results are illustrated in Table 7. The cosmetics according to Examples are excellent in use feeling both during and after application. However, the cosmetics of Comparative Examples are not good in use feeling.

**[Table 6]**

| | Cosmetic com ponents constituting pow der foundation | Blending ratio (wt% ) |
|---|---|---|
| (1) | Pow der of Exam ple or Com parative Exam ple | 10.0 |
| (2) | Sericite (silicon-treated) | 40.0 |
| (3) | Talc (silicon-treated) | 29.0 |
| (4) | Mica (silicon-treated) | 5.0 |
| (5) | Titanium oxide (silicon-treated) | 7.0 |
| (6) | Yellow iron oxide (silicon-treated) | 1.2 |
| (7) | Red iron oxide (silicon-treated) | 0.4 |
| (8) | Black iron oxide (silicon-treated) | 0.2 |
| (9) | Methylparaben | 0.2 |
| (10) | Dimethicone | 4.0 |
| (11) | Liquid paraffin | 2.0 |
| (12) | Glyceryltri-2-ethylhexanoate | 1.0 |

**[Table 7]**

| Evaluation sample | During application | | | After application | | |
|---|---|---|---|---|---|---|
| | Uniform spread | Moist feeling | Smoothness | Uniformty of film | Moist feeling | Softness |
| Example 1 (Cosmetic 1) | Excellent | Good | Good | Good | Good | Excellent |
| Example 2 (Cosmetic 2) | Excellent | Good | Good | Good | Good | Excellent |
| Example 3 (Cosmetic 3) | Good | Excellent | Excellent | Good | Excellent | Good |
| Example 4 (Cosmetic 4) | Excellent | Poor | Good | Good | Poor | Fair |
| Example 5 (Cosmetic 5) | Excellent | Good | Good | Good | Good | Excellent |
| Exampe 6 (Cosmetic 6) | Excellent | Good | Good | Good | Good | Excellent |
| Example 7 (Cosmetic 7) | Excellent | Good | Good | Good | Good | Good |
| Comparative Example 1 (Cosmetic a) | Good | Fair | Good | Good | Good | Good |
| Comparative Example 2 (Cosmetic b) | Very Poor | Excellent | Poor | Very Poor | Excellent | Very Poor |
| Comparative Example 3 (Cosmetic c) | Very Poor | Poor | Very Poor | Very Poor | Poor | Poor |

## Claims

1. Coated particles comprising starch particles surface-treated with rice bran wax, wherein
an average particle diameter d₁ is 0.5 to 20 µm, and a maximum particle diameter d₂ is less than 30 µm and 4.0 times or less the average particle diameter d₁, and
the rice bran wax is contained in an amount of 0.5 to 10.0 wt%.

2. The coated particles according to claim 1, wherein the starch particles contain 90 wt% or more of amylopectin.

3. The coated particles according to claim 1 or 2, wherein a content of globulin is 0.02 wt% or less.

4. The coated particles according to any one of claims 1 to 3, wherein a sphericity is 0.85 or more.

5. A method for producing coated particles, comprising:
a first step of preparing a dispersion that contains 1 to 20 wt% of starch particles;
a second step of adding rice bran wax to the dispersion, heating the mixture, and cooling the mixture to precipitate the rice bran wax on a surface of the starch particles; and
a third step of subjecting the dispersion obtained in the second step to solid-liquid separation to obtain coated particles as a solid.

6. The method for producing coated particles according to claim 5, wherein in the second step, 0.5 to 11.0% by mass of the rice bran wax is added with respect to a mass of the starch particles contained in the dispersion.

7. Cosmetics in which the coated particles according to any one of claims 1 to 4 are blended.
